# EUROPEAN PATENT SPECIFICATION

(11) **EP 3 008 196 B1**
(45) Date of publication and mention of the grant of the patent: **17.11.2021**
(21) Application number: 14810426.8
(22) Date of filing: 14.06.2014
(51) Int. Cl.: C12P 7/06, C12P 7/10, C12P 19/02, C12P 19/14, C08H 8/00

(54) **METHODS TO ENHANCE MICROBIAL CONVERSION OF CELLULOSIC BIOMASS WITH MECHANICAL AUGMENTATION**
VERFAHREN ZUR VERBESSERUNG DER MIKROBIELLEN UMWANDLUNG VON CELLULOSEHALTIGER BIOMASSE MIT MECHANISCHER VERSTÄRKUNG
PROCÉDÉS POUR AMÉLIORER LA CONVERSION MICROBIENNE DE BIOMASSE CELLULOSIQUE AVEC AUGMENTATION MÉCANIQUE

(30) Priority: 14.06.2013 US 201361835447 P
(43) Date of publication of application: 20.04.2016
(73) Proprietor: The Trustees Of Dartmouth College, Hanover, NH 03755 (US); Lynd, Lee R., Meriden, NH 03770 (US); Paye, Julie, M.D., Randolph Center, VT 05061 (US)
(72) Inventor: LYND, Lee, R., Meriden, NH 03770 (US); PAYE, Julie, M.D., Vermont 05061 (US)
(74) Representative: Abel & Imray
(86) International application number: PCT/US2014/042432
(87) International publication number: WO 2014/201439

(56) References cited:
- WO-A2-2010/073083
- WO-A2-2012/083244
- US-A1- 2011 143 412
- US-A1- 2012 028 325
- MARTIN J. NEILSON ET AL: "Enhancement of enzymatic hydrolysis by simultaneous attrition of cellulosic substrates", BIOTECHNOLOGY AND BIOENGINEERING., vol. 24, no. 2, February 1982 (1982-02), pages 293-304, XP055327520, US ISSN: 0006-3592, DOI: 10.1002/bit.260240204
- DETROY ET AL.: 'Biomass conversion: fermentation chemicals and fuels' CRITICAL REVIEWS IN MLCROBIOLOGY vol. 10, no. ISSUE, February 1983, pages 203 - 228, XP055300842
- ARCHAMBAULT-LEGER ET AL.: 'Integrated analysis of hydrothermal flow through pretreatment' BIOTECHNOLOGY FOR BIOFUELS vol. 5, no. 49, 19 July 2012, pages 1 - 10, XP021122736
- None

## Description

### RELATED APPLICATION

### BACKGROUND

### I. Field of the Invention

The disclosure relates to conversion of biomass to biofuel or other useful products. More particularly, the disclosure pertains to the use of microorganisms and mechanical force to enhance biomass conversion.

### II. Description of the Related Art

Biomass is a relatively inexpensive, renewable and abundant material that can be used to generate fuels, chemicals, fibers, and energy. However, large-scale utilization of plant biomass is hindered, at least in part, by the lack of technologies capable of efficiently converting the biomass into component fractions or reactive intermediates at a low cost. For example, most plant biomass is resistant to digestion by cellulase.

Pretreatment of biomass may render the biomass more amenable to enzymatic digestion. Pretreatment may remove biomass components such as lignin and/or hemicellulose that impede access to cellulase enzymes. Pretreatment may also cause structural changes (e.g. particle size, porosity, surface area) in the biomass. Various biomass pretreatment technologies have been developed. Examples of these developments include use of dilute acids or bases, steam explosion, autohydrolyisis, controlled pH, AFEX, and aqueous ammonia pretreatment.

Based on results gleaned from studies using woody feedstocks and fungal cellulases, it is commonly believed that pretreatment of cellulosic biomass is required for all biomass. According to this understanding, pretreatment using high temperature and/or chemicals is necessary prior to biological processing in order to achieve economically viable yields upon subsequent enzymatic hydrolysis.

However, pretreatment adds to the cost and may affect performance during subsequent fermentation. Capital and operating costs for pretreatment are substantial, and pretreatment further increases processing costs by negatively impacting the performance of downstream processing operations. In particular, all known pretreatments either produce compounds which inhibit hydrolysis and fermentation or require recovery of chemical reagents (for example, ammonia or ionic liquids), or both.

US2012/028325 discloses a method for converting lignocellulose containing biomass into ethanol or other products wherein the biomass can be mechanically pretreated e.g. by ball-milling. Pretreatment is required to reduce the lignin content, reduce the order of the cellulose and increases surface area.

R. Detroy et al.: "Biomass conversion: fermentation chemicals and fuels", Critical Reviews in Microbiology, vol. 10, February 1983, pages 203-228, teaches mechanical disruption of lignocellulosic biomass by milling prior to fermentation.

V. Archambault-Leger et al.: "Integrated analysis of hydrothermal flow through pretreatment", Biotechnology for Biofuels, vol. 5, no. 49, 19 July 2012, pages 1-10 teaches that ball milling prior to hydrolysis substantially accelerated hydrolysis for SSF processes and a method performed with C. thermocellum.

WO2010/073083 discloses a method for producing ethanol from ensiled lignocellulosic biomass comprising mechanically pretreating the lignocellulosic biomass prior to ensiling and subjecting the biomass to enzymatic hydrolysis.

WO2012/083244 discloses a method for producing ethanol by mechanically treating biomass prior to fermentation. The mechanical treatment (shredding, milling, chipping, crushing, grinding, or pulverizing) leads to biomass particle size reduction. The microorganisms present in the reactor are Clostridia thermocellum, C. cellulolyticum, C. polysaccharolyticum, Thermoanaerobacterium saccharolyticum and T. thermosaccharolyticum.

M. J. Neilson et al.: "Enhancement of enzymatic hydrolysis by simultaneous attrition of cellulosic substrates", Biotechnology and Bioengineering, vol. 24, no. 2, February 1982, pages 293-304, teaches mechanical pretreatment of lignocellulosic material by ball milling prior to fermentation.

### SUMMARY

The invention discloses a method for converting biomass efficiently. High solubilization rates of cellulosic feedstocks may be achieved with little or no pretreatment by using cellulolytic microbes in conjunction with mechanical disruption of lignocellulose particles during the conversion process.

The cellulosic biofuel field has operated based on the understanding that pretreatment is required for all biomass. It is disclosed here that such pretreatment step may be eliminated. For purpose of this disclosure, the term "pretreatment" refers to the process of contacting biomass with any chemicals other than water. For purpose of this disclosure, the term "pretreatment" does not include autoclaving.

It is disclosed here a method for converting biomass into ethanol or other desired products. The biomass comprises lignocellulosic particles. In one aspect, the biomass has not been pretreated. In another aspect, the biomass is not pretreated with any chemicals. In another aspect, the biomass has been in contact with water only prior to the treatment. In another aspect, the biomass has been autoclaved before being loaded into the reactor. In another aspect, the reactor is a closed reactor.

Also disclosed is a method for converting biomass into ethanol or other desired products. The method includes (a) adding the biomass and a microorganism to a reactor, wherein the microorganism is capable of solubilizing the biomass and fermenting the solubilization products. The biomass contains lignocellulosic particles.

The method of the invention includes (a) adding a biomass and a microorganism to a reactor, said biomass comprising lignocellulosic particles, (b) fermenting the biomass with the microorganism to form a fermented product, said fermented product comprising the microorganism, fermented biomass, and lignocellulosic particles, (c) mechanically disrupting the first fermented product of step (b), wherein the mechanical disrupting comprises mechanical milling, and (d) fermenting the mechanically disrupted product of step (c), wherein said steps (b)-(d) are repeated N times, N being an integer equal to or greater than 1, said microorganism being at least one member selected from the group consisting of *Clostridium thermocellum, Clostridium claraflavum, Caldicellusiruptor bescii, Thermoanaerobacterium saccharolyticum, Thermoanaerobacterium thermosaccharolyticum.* N may be in the range of from 1 to 10.

The milling of fermented solids may be carried out at about 8%, 10%, 12% or 15% (w/w) of solids.

More than 50%, 60%, 65%, 70%, 75%, or 80% of sugar in the biomass may be solubilized after 5-day with incubation with intermittent mechanical disruption.

One or more microorganisms may be used and the microorganisms are capable of fermenting the biomass to desired products. The microorganisms are capable of solubilizing the biomass and fermenting the solubilization products. The microbial system as disclosed herein include pure culture or co-culture of microorganisms such as *Clostridium thermocellum, Clostridium claraflavum, Caldicellusiruptor bescii, Thermoanaerobacterium saccharolyticum, Thermoanaerobacterium thermosaccharolyticum,* or combination thereof. By way of example, strains that may be used in the microbial system include but are not limited to *Clostridium thermocellum* DSM1313, *Clostridium thermocellum* ATCC 27405, *Clostridium claraflavum* 42A, *Clostridium claraflavum* DSM19732, among others.

The microbial fermentation and the mechanical disruption of the cellulosic material occur sequentially during fermentation.

A system including a powering means for using pressure developed in the reactor to power the means for mechanical disruption may be used. The pressure may be from gas generated by fermentation in the reactor, or it may come from static pressure developed at the bottom of the fermentor (or reactor). For instance, production of CO2 by fermenting organisms may be contained which may result in a transient increase in pressure. This increase in pressure may be used as a motive force to propel cellulosic slurries through an orifice (e.g. from one reactor to another, as in a cascade continuous configuration), or alternatively may be used to increase the pressure of the fermentation system (or portion thereof).

Mechanical disruption of lignocellulosic particles may be achieved by a number of different ways. By way of example, the disruption may be accomplished by using solid particles (e.g. metal spheres) with density higher than water in the reactor (or fermentor). The disruption may be accomplished by exposure to sheer by intense mixing, and optionally additionally by passage through an orifice or nozzle. The disruption may be accomplished by sending lignocellulosic particles through a mill (e.g. disc refiner) outside of the fermentor and recycling them back to the fermentor after the mechanical processing. The means for mechanically disruption and the powering means may include a nozzle or a hydrocyclone with dense beads.

The means for mechanical disruption may include ball mill, disc refiner or both. Metal or glass balls having a uniform diameter may be used. Metal balls having different diameters may be used together to enhance the disruption. By way of example, metal balls having different diameters ranging from 2mm to 50mm, from 5mm to 30 mm, or from 8mm to 20mm may be used.

### BRIEF DESCRIPTION OF THE DRAWINGS

Figure 1 is a flow chart showing the different routes of the combined mechanical and biochemical treatment process.
Figure 2 shows glucan solubilization results after Round 1 and Round 2 for each of the two Routes A and B, respectively. The Cumulative glucan solubilization is also shown in Fig. 2.
Figure 3 shows solubilization of glucan and xylan in switchgrass by SSF featuring fungal cellulase hydrolysis and by microbial conversions using various microbial systems.
Figure 4 shows the high conversions achieved by bacterial/microbial conversion and enhanced by mechanical force on grass (washed or unwashed) compared to the much lower conversions obtained from SSF featuring added fungal cellulase on wood or grass.
Figure 5 shows results comparing solubilization of the switchgrass after 5-day treatment with different microorganisms.
Figure 6 shows the various products from the solubilization and conversion experiments described in Fig. 5.
Figurer 7 shows comparative glucan solubilization results of autoclaved but not otherwise pretreated mid-June harvested switchgrass and poplar by C. thermocellum and by SSF using fungal cellulase and yeast.
Figure 8 shows the impact of reduced solids loading on solubilization.
Figure 9 shows the effects of harvest date, choice of biocatalyst, and particle size on glucan solubilization for washed, autoclaved but not otherwise pretreated feedstocks.
Figure 10 shows comparative glucan solubilization from autoclaved but not otherwise pretreated mid June-harvested switchgrass by various loadings of C. thermocellum enzymes or fungal cellulase after 5 days in serum vials.
Figure 11 is a plot showing glucan and xylan solubilization for multiple substrates and conversion systems.
Figure 12 shows the predicted and actual solubilization for multiple substrates and conversion systems.
Figure 13 shows the impact of ball-milling on successive fermentations of autoclaved but not otherwise pretreated October-harvested switchgrass.

### DETAILED DESCRIPTION

It is commonly believed in the field of cellulosic biofuel that pretreatment is required for effective conversion of cellulosic biomass to ethanol. Pretreatment adds to the cost and may affect performance during subsequent fermentation. Capital and operating costs for pretreatment are substantial, and pretreatment further increases processing costs by negatively impacting the performance of downstream processing operations. In particular, all known pretreatments either produce compounds which inhibit hydrolysis and fermentation or require recovery of chemical reagents (e.g. ammonia or ionic liquids), or both.

In addition to cost benefit, processing biomass without pretreatment may also foster recovery of co-products such as feed protein and high molecular weight lignin, and may make biomass processing substantially less hazardous. If cellulosic biomass can be processed with high yields and reasonably high rates without pretreatment, this would likely be a revolutionary advance resulting in processes that are much less expensive, simpler, and more reliable. Such an advance has clear potential to preempt other technical approaches and be of tremendous competitive advantage.

Potential disadvantage of biomass conversion with little or no pretreatment may include, for example, uncertainty about the impact of feedstock characteristics on amenability to processing, the possibility that solubilization will not be as effective at high concentrations, and that thermophiles cannot be engineered to achieve high ethanol yields and titers. It is disclosed here that high solubilization rates of cellulosic feedstocks may be achieved with little or no pretreatment by using cellulolytic microbes in conjunction with mechanical disruption of lignocellulose particles during the conversion process.

The combination of microbial cellulose utilization with mechanical disruption of lignocellulose particles has not been shown for the production of fuels or chemicals from cellulosic biomass. Physical disruption during the conversion process has been demonstrated. *See, e.g.* Ryu and Lee, Biotechnol Bioeng. 1983, 25(1):53-65; Jones and Lee, Biotechnol Bioeng. 1988, 31(1):35-40; Tjerneld et al., Bioseparation. 1990, 1(3-4):255-63. However, those studies all used cell-free enzymatic hydrolysis, rather than the microbial system as disclosed herein. The present disclosure shows the advantage of microbial conversion in conjunction with mechanical force over cell-free enzymatic conversion.

All prior studies used an attrition reactor. Certain limitations have been observed in studies of enzymatic hydrolysis in an attrition reactor. These limitations include, by way of example, decreased effectiveness at high solids, accelerated enzyme inactivation, among others. These limitations associated with cell-free enzymatic hydrolysis in an attrition reactor may be less important in a microbial system because of the different mechanisms involved in the two systems.

Combined microbial conversion and mechanical disruption is likely to be both less expensive and more effective as compared to milling prior to conversion because of (a) the softening of the particles during conversion, which is expected to make them considerably easier to disrupt as compared to particle size reduction prior to conversion, and (b) the generation of new surfaces during the reaction process.

The term "biomass" refers to non-fossilized renewable materials that are derived from or produced by living organisms. In its broadest term, biomass may include animal biomass, plant biomass, and human waste and recycled materials, among others. Examples of animal biomass may include animal by-product and animal waste, etc. In this disclosure, biomass refers to plant biomass which includes any plantderived matter (woody or non-woody) that is available on a sustainable basis. Plant biomass may include, but is not limited to, agricultural crop wastes and residues such as corn stover, wheat straw, rice straw, sugar cane bagasse and the like, grass crops, such as switch grass, alfalfa, winter rye, and the like. Plant biomass may further include, but is not limited to, woody energy crops, wood wastes and residues such as trees, softwood forest thinnings, barky wastes, sawdust, paper and pulp industry residues or waste streams, wood fiber, and the like. In urban areas, plant biomass may include yard waste, such as grass clippings, leaves, tree clippings, brush, etc., vegetable processing waste, as well as recycled cardboard and paper products.

Grassy biomass may be used in the present disclosure. Winter cover crops such as winter rye may be used as a bioenergy feedstock using existing equipment and knowhow. Winter cover crops have little and arguably no competition with food crops for land or revenue, and they also positively impact soil and water quality as well as farm income, and offer important co-product opportunities. A recent study estimated that 200 million dry tons of winter rye per year could be produced in the U.S. on land used to grow corn and soybeans, which has a liquid fuel production potential equal to that of the current U.S. and Brazilian industries combined.

By using the methods disclosed herein, other cellulosic feedstocks may also be processed into biofuels without pretreatment. The microorgamisms are selected from at least one member of the group consisting of *C. thermocellum, C. clarafalvum, C. bescii or C. thermocellum*/*Thermoanaerobacterium sαccharolyticum* co-culture as fermentation systems. Various strategies for mechanical augmentation may be employed to further enhance the conversion. These strategies may include, by way of example, *in situ* ball-milling, passage through a nozzle or a high-sheer in-line mixer, and several configurations involving use of pressure developed during fermentation as a motive force. The use of microbial cellulose fermentation and mechanical means simultaneously may prove to be an cost-effective and efficient way for enhancing biomass conversion.

Having now described certain embodiments in detail, the same will be more clearly understood by reference to the following examples, which are included for purposes of illustration only and are not intended to be limiting.

### Examples

### Example 1 Mechanical disruption contributed to higher solubilization rates

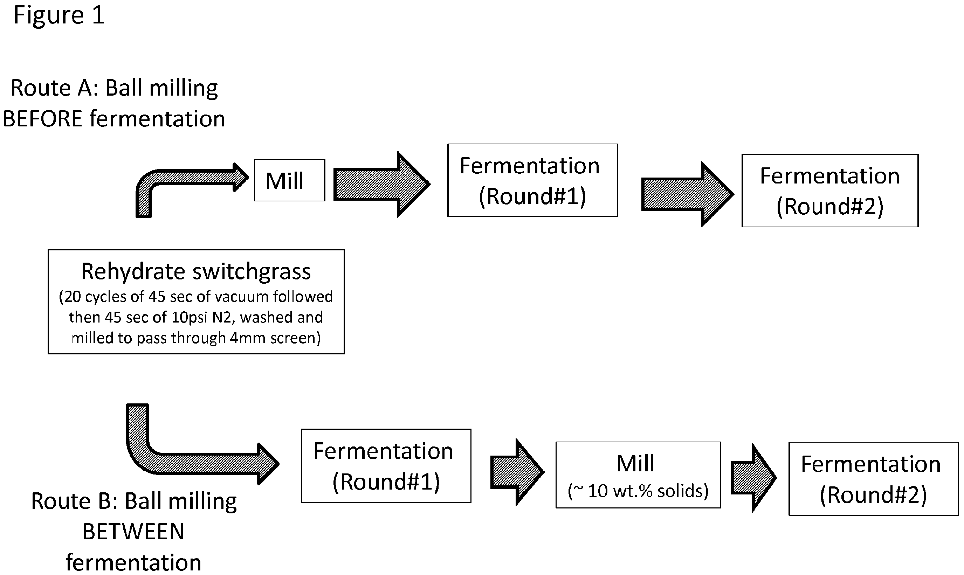

Fig. 1 is a flow chart showing the different routes of the combined mechanical and biochemical treatment process. In Route A, not according to the invention, the biomass is subject to ball milling before fermentation. In Route B, the biomass is subject to ball milling between the two Rounds of fermentations.

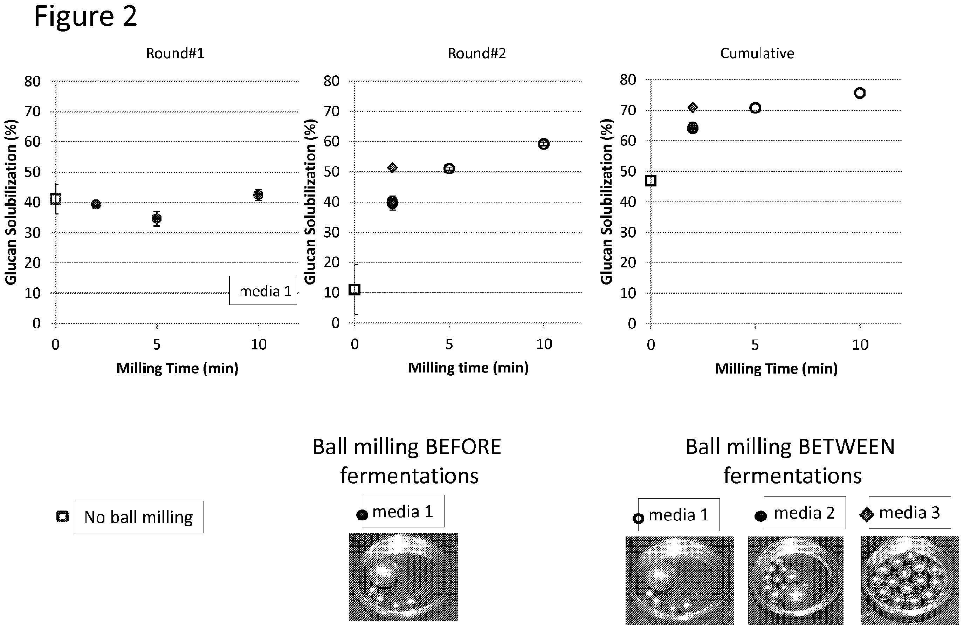

Fig. 2 shows glucan solubilization results after Round 1 and Round 2 for each of the two Routes A and B, respectively. The cumulative glucan solubilization is also shown in Fig. 2. The diameters of the balls in media 1 and 2 were 8mm, 11mm, and 20mm. The diameter of the balls in media 3 was 11mm.

### Example 2 Comparing solubilization results of SSF and various microorganisms

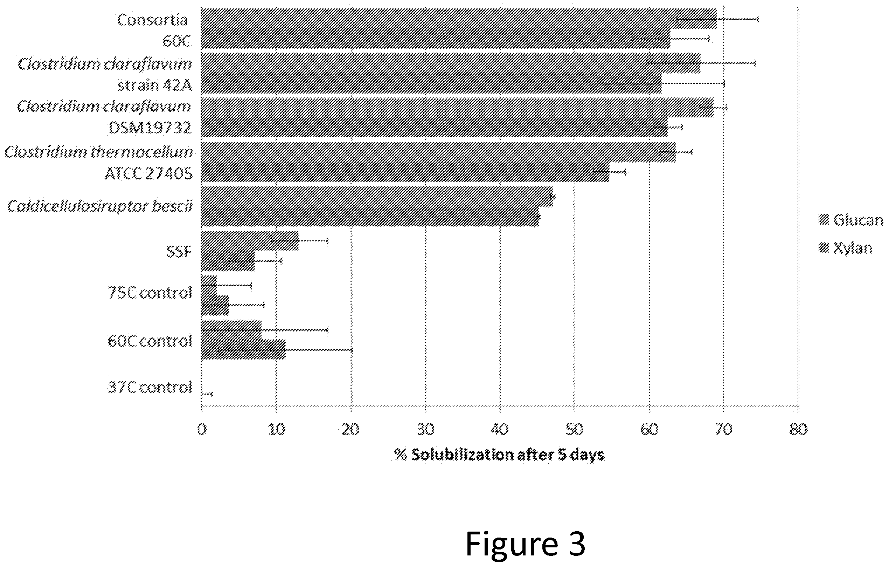

Experiments were conducted to compare the solubilization results of SSF and various microorganisms. Fig. 3 shows solubilization of glucan and xylan in switchgrass by Simultaneous Saccharificiaton and Fermentation (SSF) featuring fungal cellulase hydrolysis and microbial conversions using various microbial systems. While SSF achieved 13% solubilization of cellulose after 5 days, solubilization by most microbial systems tested exceeded 60% solubilization.

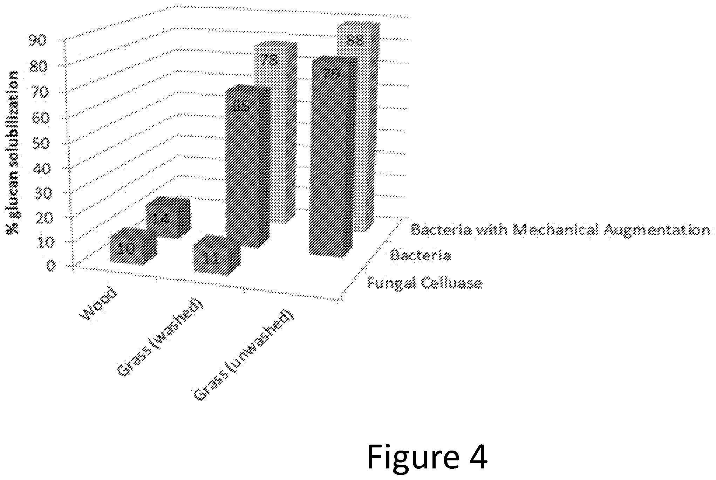

Fig. 4 highlights the differences between enzymatic and microbial conversion on untreated woody versus grassy lignocellulose. While performances of fungal cellulases were similar between untreated wood and grass (10% vs 11%), microbial conversions of untreated grass were over 4 times greater on grass than wood (65% vs 14%). Conversion was further increased to 78% by introducing a means of mechanical disruption/augmentation. The results labeled "washed" were performed on switchgrass in which soluble glucans were removed (60 C overnight wash). Glucan solubilization for bacteria with mechanical augmentation was 88% for unwashed switchgrass (Fig. 4).

As shown in Figs. 3 and 4, microbial cellulose conversion shows superior hydrolysis performance on untreated or minimally-pretreated substrates when compared to conventional systems using fungal cellulases or cell-free cellulase (Fig 3). Microbial conversion combined with mechanical disruption during conversion may be more effective than microbial conversion without mechanical force (or disruption). As shown in Fig. 4, microbial conversion may achieve about 65% conversion or greater in the absence of mechanical disruption. Higher extents of solubilization may be achieved when mechanical disruption is employed.

While solubilization yields for un-pretreated switchgrass typically need to be increased by greater than 5-fold in order to achieve the high extents of solubilization generally required for attractive economics, an increase of only 30% or so in a microbial cellulose system may be sufficiently cost-effective. Thus, while a relatively small intensity of mechanical disruption may be required for microbial conversion, much more severe (or harsh) disruption may be required for conversion of unpretreated substrates using fungal cellulases.

### Example 3 Comparing solubilization results of various microorganisms

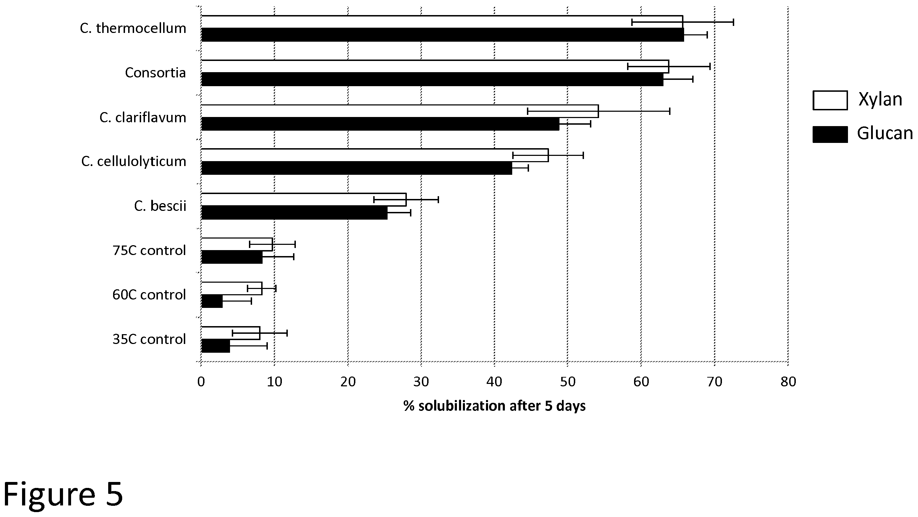

Solubilization results using various feedstocks (switchgrass and poplar) and conversion systems (C. *therm* & SSF with fungal cellulase, C. *bescii, C. clariflavum, C. cellulolyticum,* mixed enrichment) were examined and compared. Solubilization of autoclaved but not otherwise pretreated washed switchgrass harvested in mid-June was examined using a number of microorganisms. Fig. 5 shows the comparative results comparing solubilization of the switchgrass after 5-day treatment with different microorganisms. Parameters for these tests were: 5 g/L glucan, 13 g/L solid, < 4mm particle size, 2% inoculum after 5 days in serum vials. The consortia were enriched at 60 °C on Avicel from horse manure compost. Results are expressed as mean +/- standard deviation for three independent tests each carried out in triplicate. As shown in Fig. 5, C. *thermocellum* and the consortia both showed about 65% solubilization, while other microorganisms tested showed lower solubilization percentages.

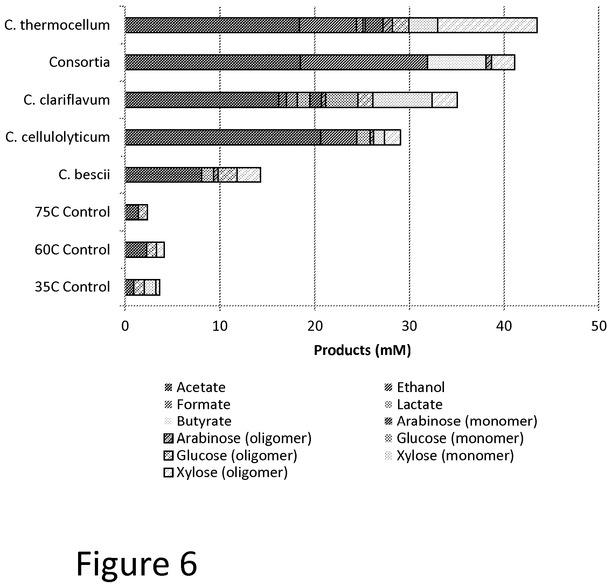

Fig. 6 shows the solubilization products for the experiments in Fig. 5. Fermentation and hydrolysis products from washed green switchgrass (4mm particle size) by various microorganisms (2% inoculum) after 5 days. Consortia were enriched on Avicel from horse manure compost. Results are expressed as mean (n=3). As shown in Fig. 6, acetate was present at highest concentrations for all cultures and controls. Soluble sugars were present at significant levels for all cultures except the consortium (no pH control).

### Example 4 Comparing solubilization results of different biomass using different microorganisms and SSF

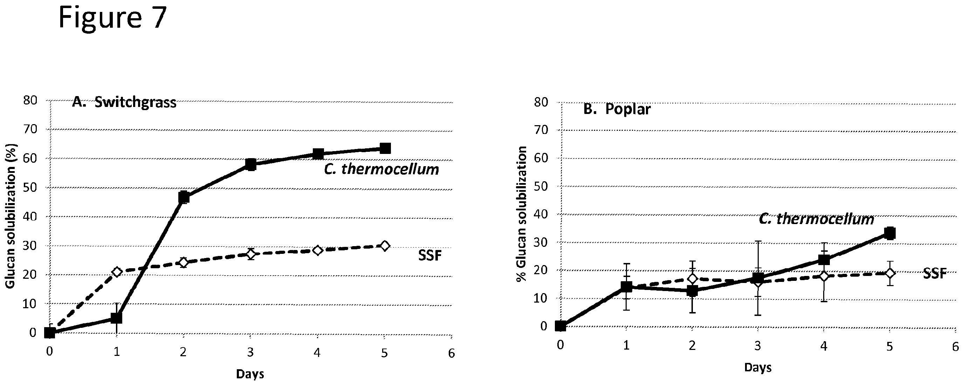

Glucan solubilization of autoclaved but not otherwise pretreated mid-June harvested switchgrass and poplar by different microorganisms or cellulase was examined. Results of C. thermocellum and SSF using fungal cellulase and yeast were shown in Fig. 7. The tests were conducted in serum vials for 5-day incubation. Fungal cellulase loadings were 4.5 mg Ctec2 per gram solid and 0.5 mg Htec2 per gram solid. Solubilization of uninoculated switchgrass (5 g/L glucan, 13 g/L solid, <4mm particle size) and poplar (5 g/L glucan, 11 g/L solid, <0.5mm particle size) was less than 10%. Results are expressed as mean +/- standard deviation for three independent experiments each carried out in triplicate. As shown in Fig. 7, *C*. thermocellum conversion exceeded SSF by about 2-folds at low enzyme loading, and mid-June switchgrass was much more reactive than wood for both systems.

### Example 5 Effects of the amount of solid loading on solubilization

The impact of reduced solids loading on solubilization was studied. Glucan solubilization from washed mid June-harvested switchgrass with an initial solids concentration of 13 g/L or 2.5 g/L after 5 days was compared. Fungal cellulase loading was 4.5 mg Ctec2/ g solid and 0.5 mg Htec2 / g solid. Results are expressed as mean +/- standard deviation for three independent experiments each carried out in triplicate.

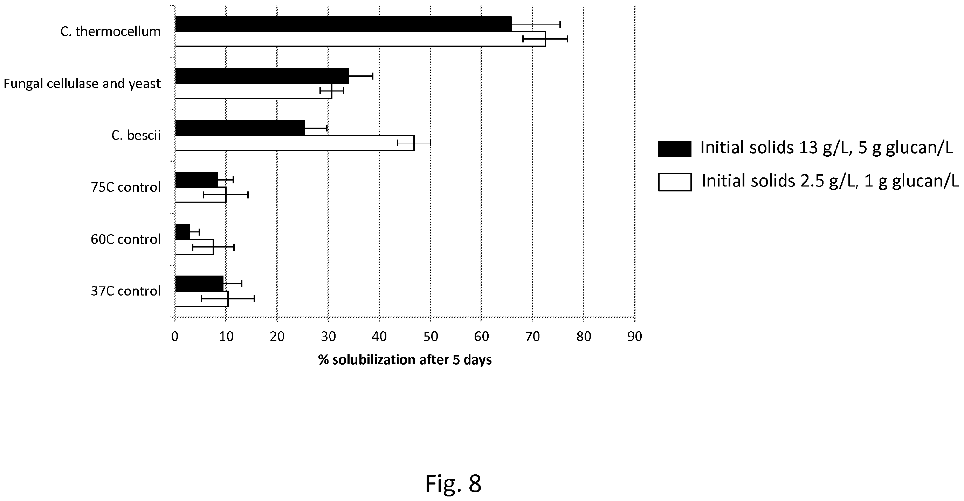

As shown in Fig. 8, initial solids loading had a significant impact on glucan solubilization for C. *bescii,* but not for C. *thermocellum* or SSF.

### Example 6 Effects of biomass harvest date and particle size on solubilization

Glucan solubilization for washed, autoclaved but not otherwise pretreated feedstocks as a function of harvest switchgrass harvest date, choice of biocatalyst, and particle size was studied. C. thermocellum (filled squares) or fungal cellulase and yeast (open diamonds), with 5 day incubation in serum vials was used in the study. Fungal cellulase loading was 4.5 mg Ctec2/ g solid and 0.5 mg Htec2 / g solid. Solubilization of uninoculated young switchgrass (5 g/L glucan, 13 g/L solid), senescent switchgrass (5 g/L glucan, 12 g/L solid) and poplar (5 g/L glucan, 11 g/L solid) was less than 10%. Results are expressed as mean +/- standard deviation for three independent experiments each carried out in triplicate.

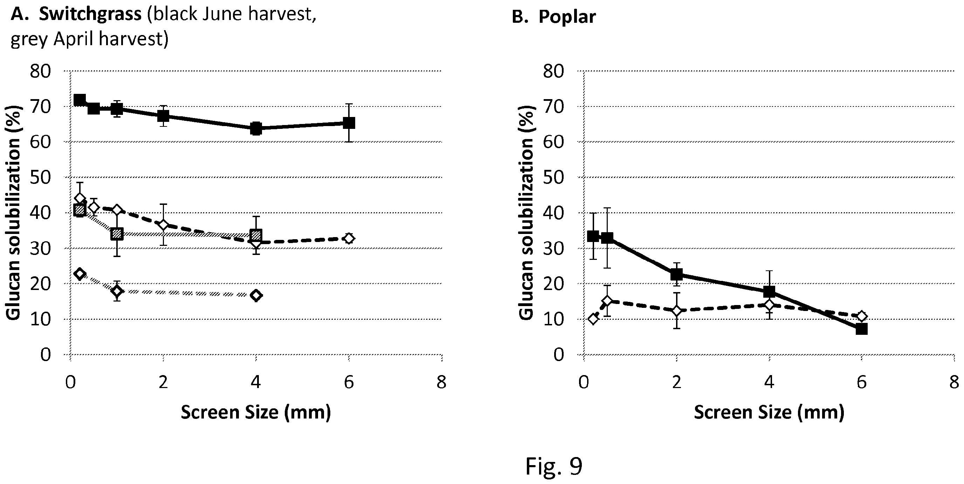

As shown in Fig. 9, particle size did not show a significant impact for grass, poplar SSF. However, particle size appeared to have some significant impact for C. thermocellum on poplar.

Solubilization of senescent switchgrass was about half that of mid-June harvested switchgrass for both C. thermocellum and SSF, suggesting that harvest date also has a significant impact on solubilization. Here again, C. thermocellum was about 2 times as effective as SSF on swtichgrass at all particle sizes and at both maturities.

### Example 7 Effects of enzyme loading and source of enzymes on solubilization

Comparative glucan solubilization from autoclaved but not otherwise pretreated mid June-harvested switchgrass by various loadings of C. thermocellum enzymes or fungal cellulase after 5 days in serum vials. 5 g/L glucan, 13 g/L solid, 4mm particle size.

C. thermocellum enzymes were purified by either affinity purification (∘) or by concentrating and dialyzing cell-free broth (•). Fungal cellulase was incubated at 37 C in the presence (X) or absence of yeast (□), at lower substrate concentration (Δ, 2.5 g/L solids ), or increased hydrolysis temperature (◊, 50 C). Results are expressed as mean +/- standard deviation (n=3 for fungal cellulase, n=2 for affinity purified cellulase, n=1 for dialyzed concentrated broth).

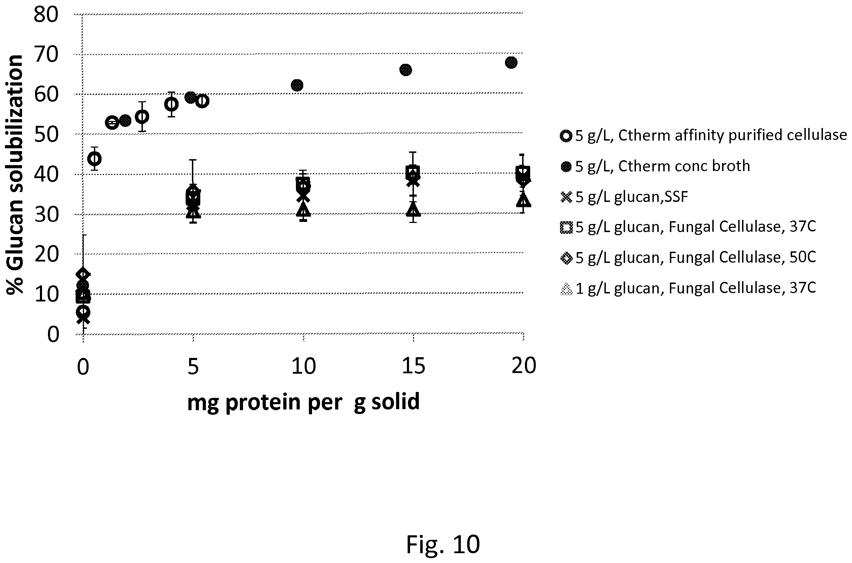

As shown in Fig. 10, 5 day SSF solubilization was not significantly improved by the presence or absence of yeast, higher enzyme loadings, higher incubation temperature, or lower feedstock loading. *C therm* cellulase was much more effective than fungal cellulase at all loadings.

### Example 8 Glucan and xylan solubilization for multiple substrates and conversion systems

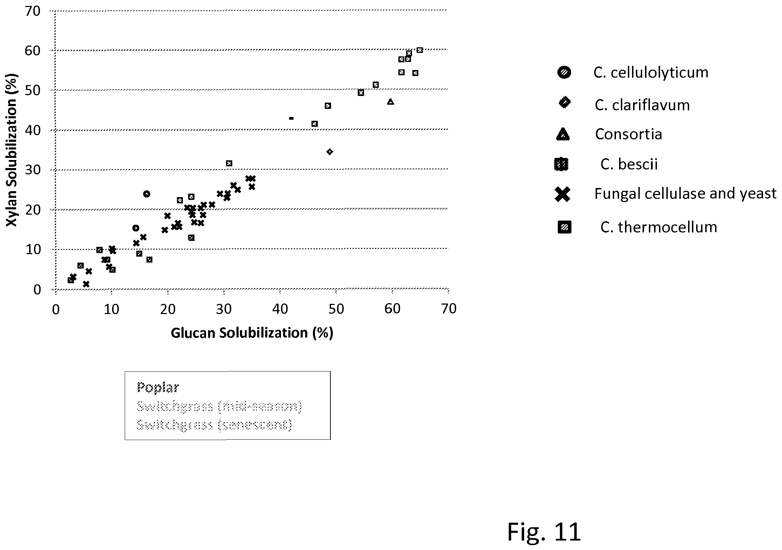

Glucan and xylan solubilization for multiple substrates by conversion systems were examined. Fig. 11 shows the glucan and xylan solubilization for multiple substrates and conversion systems.

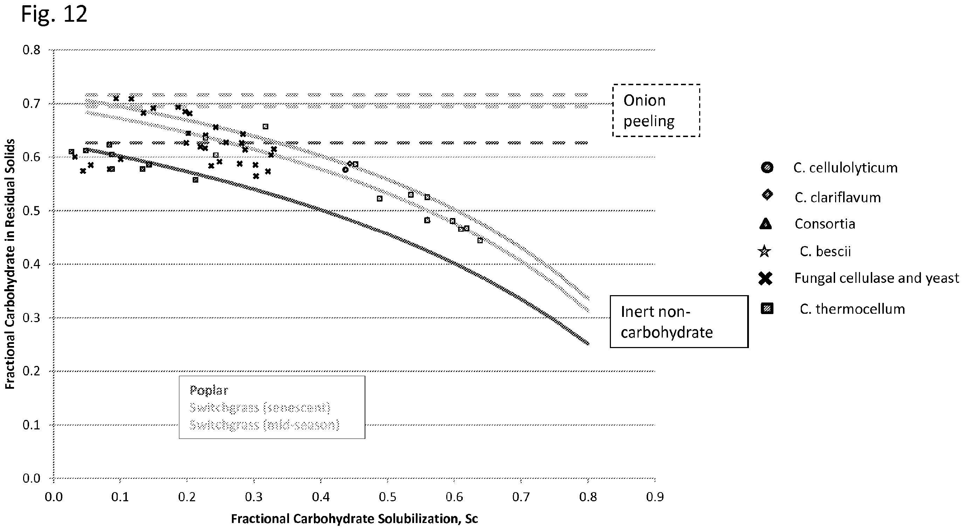

Fig. 12 shows the predicted and actual solubilization for multiple substrates and conversion systems. The data obtained from 3 substrates and 6 conversion systems confirms inert non-carbohydrate hypothesis, while rejecting the "onion peeling" hypothesis, which suggests that non-carbohydrate is solubilized to the same extent as carbohydrate.

### Example 9 Impact of ball-milling on successive fermentations

The effects of ball-milling on successive fermentations of autoclaved but not otherwise pretreated October-harvested switchgrass were studied. The term "before" refers to milling before the first inoculation, and the term "after" refers to milling between the first and second inoculation.

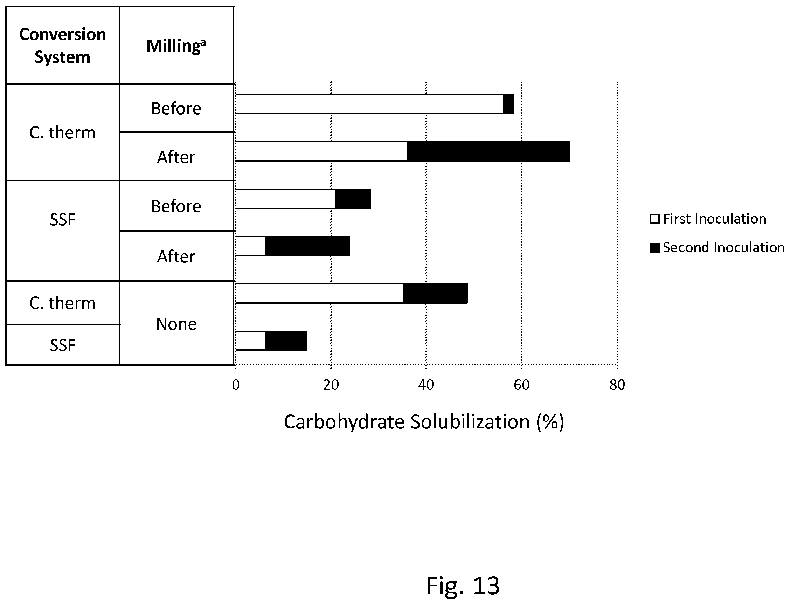

As shown in Fig. 13, in the case of C. *thermocellum,* five minutes of milling after the first fermentation but before the second fermentation resulted in increased solubilization of the remaining carbohydrate during the second fermentation phase. More specifically, with the milling between the first fermentation and the second fermentation, greater than 50% of the remaining carbohydrate after the first fermentation was solubilized. The same milling before the first fermentation had no effect as compared to the no-milling controls. Thus, physical and biological attack are much more effective when done alternately (co-treatment) rather than sequentially (pretreatment).

High solubilization of senescent, autoclaved but otherwise unpretreated switchgrass may be achieved by C. *thermocellum* with brief milling, but most likely not by SSF with the same milling treatment.

## Claims

1. A method for converting biomass into ethanol or other products, said method comprising
(a) adding a biomass and a microorganism to a reactor, said biomass comprising lignocellulosic particles,
(b) fermenting the biomass with the microorganism to form a fermented product, said fermented product comprising the microorganism, fermented biomass, and lignocellulosic particles,
(c) mechanically disrupting the first fermented product of step (b), wherein the mechanical disrupting comprises mechanical milling, and
(d) fermenting the mechanically disrupted product of step (c),
wherein said steps (b)-(d) are repeated N times, N being an integer equal to or greater than 1 said microorganism being at least one member selected from the group consisting of *Clostridium thermocellum, Clostridium claraflavum, Caldicellusiruptor bescii*, *Thermoanaerobacterium saccharolyticum, Thermoanaerobacterium thermosaccharolyticum.*

2. The method of claim 1, wherein said step (c) may be accomplished by sending lignocellulosic particles of said first fermented product through a mill outside of the fermentor and recycling said lignocellulosic particles back to the fermentor after mechanical processing.

3. The method of claim 1, wherein said step (c) is performed by mechanically disrupting said biomass with balls or beads made of metal or glass.

4. The method of claim 1, wherein said reactor is a closed reactor.

## Patentansprüche

1. Verfahren zur Umwandlung von Biomasse in Ethanol oder andere Produkte, wobei das Verfahren umfasst
(a) Zugeben von Biomasse und eines Mikroorganismus in einen Reaktor, wobei die Biomasse Lignocellulose-Partikel umfasst,
(b) Fermentieren der Biomasse mit dem Mikroorganismus zur Bildung eines fermentierten Produkts, wobei das fermentierte Produkt den Mikroorganismus, fermentierte Biomasse und Lignocellulose-Partikel umfasst,
(c) mechanisches Zerstören des ersten fermentierten Produkts nach Schritt (b), wobei das mechanische Zerstören ein mechanisches Zerkleinern umfasst, und
(d) Fermentieren des mechanisch zerstörten Produkts nach Schritt (c), wobei die Schritte (b)-(d) N Mal wiederholt werden, und N eine ganze Zahl gleich oder größer als 1 ist,
wobei der Mikroorganismus mindestens ein Glied ist, ausgewählt aus der Gruppe, bestehend aus *Clostridium thermocellum, Clostridium claraflavum, Caldicellusiruptor bescii, Thermoanaerobacterium saccharolyticum, Thermoanaerobacterium thermosaccharolyticum.*

2. Verfahren nach Anspruch 1, wobei Schritt (c) ausführbar ist durch Leiten von Lignocellulose-Partikeln des ersten fermentierten Produkts durch eine Zerkleinerungseinrichtung außerhalb des Fermenters und Rückführen der Lignocellulose-Partikel in den Fermenter nach der mechanischen Verarbeitung.

3. Verfahren nach Anspruch 1, wobei Schritt (c) durch mechanisches Zerstören der Biomasse mit Kugeln oder Perlen aus Metall oder Glas durchgeführt wird.

4. Verfahren nach Anspruch 1, wobei der Reaktor ein geschlossener Reaktor ist.

## Revendications

1. Procédé de conversion de biomasse en éthanol ou autres produits, ledit procédé comprenant
(a) l'ajout d'une biomasse et d'un micro-organisme à un réacteur, ladite biomasse comprenant des particules lignocellulosiques,
(b) la fermentation de la biomasse avec le micro-organisme pour former un produit fermenté, ledit produit fermenté comprenant le micro-organisme, la biomasse fermentée, et des particules lignocellulosiques,
(c) la désagrégation mécanique du premier produit fermenté de l'étape (b), dans lequel la désagrégation mécanique comprend le broyage mécanique, et
(d) la fermentation du produit mécaniquement désagrégé de l'étape (c),
dans lequel lesdites étapes (b) à (d) sont répétées N fois, N étant un nombre entier supérieur ou égal à 1,
ledit micro-organisme étant au moins un élément choisi dans le groupe constitué par *Clostridium thermocellum, Clostridium claraflavum, Caldicellusiruptor bescii, Thermoanaerobacterium saccharolyticum, Thermoanaerobacterium thermosaccharolyticum.*

2. Procédé selon la revendication 1, dans lequel ladite étape (c) peut être accomplie par envoi de particules lignocellulosiques dudit premier produit fermenté à travers un broyeur à l'extérieur du fermenteur et recyclage desdites particules lignocellulosiques dans le fermenteur après traitement mécanique.

3. Procédé selon la revendication 1, dans lequel ladite étape (c) est réalisée par désagrégation mécanique de ladite biomasse avec des boules ou des billes constituées de métal ou de verre.

4. Procédé selon la revendication 1, dans lequel ledit réacteur est un réacteur fermé.
